**Europäisches Patentamt**

**European Patent Office**

**Office européen des brevets**

(19)

(11) Veröffentlichungsnummer: **0 166 102**
**B1**

(12)

# EUROPÄISCHE PATENTSCHRIFT

(45) Veröffentlichungstag der Patentschrift:
**07.01.88**

(51) Int. Cl.⁴: **A 61 B 17/22**

(21) Anmeldenummer: **85104658.1**

(22) Anmeldetag: **17.04.85**

(54) Vorrichtung zur berührungsfreien Zerkleinerung von Konkrementen mit koaxialer Ortung.

(30) Priorität: **15.05.84 DE 3417985**

(43) Veröffentlichungstag der Anmeldung:
**02.01.86 Patentblatt 86/1**

(45) Bekanntmachung des Hinweises auf die Patenterteilung:
**07.01.88 Patentblatt 88/1**

(84) Benannte Vertragsstaaten:
**BE FR GB IT NL SE**

(56) Entgegenhaltungen:
**DE - A - 3 122 056**
**DE - A - 3 220 751**
**DE - B - 2 351 247**

(73) Patentinhaber: **DORNIER SYSTEM GmbH,**
**Postfach 1360, D-7990 Friedrichshafen (DE)**

(72) Erfinder: **Chaussy, Christian, Prof., 2974 Tiffany Circle,**
**Los Angeles Californien 90077 (US)**
Erfinder: **Hepp, Wolfgang, Dr., Hardtstrasse 6,**
**D-7997 Immenstaad (DE)**
Erfinder: **van Rijn, Dick, Gartenstrasse 9,**
**D-7991 Eriskirch (DE)**
Erfinder: **Forssmann, Bernd, Dr., Salemweg 6,**
**D-7990 Friedrichshafen 1 (DE)**

(74) Vertreter: **Landsmann, Ralf, Dipl.-Ing., Kleeweg 3,**
**D-7990 Friedrichshafen 1 (DE)**

## Beschreibung

Die Erfindung betrifft eine Vorrichtung zur berührungsfreien Zerkleinerung von Konkrementen in Körpern von Lebewesen nach dem Oberbergriff des Anspruchs 1.

Aus der DE-PS 2 351 247 ist eine Einrichtung zum Zerkleinern von im Körper eines Lebewesens befindlichen Konkrementen mit einer Fokussierungskammer, die Teil eines Rotationsellipsoids ist und in deren einen Brennpunkt Stosswellen durch Funkenentladung erzeugbar sind, bekannt. Damit ist eine Zerkleinerung von zum Beispiel Nierensteinen ohne operativen Eingriff oder Einführung von Sonden möglich.

In den 1983 von der DORNIER SYSTEM GMBH ausgelieferten Nierenlithotriptern wird der Nierenstein mittels zweier Röntgenabbildungssysteme in seiner Grösse und seiner Lage im Körper des Patienten lokalisiert. Dann wird der Stein von fokussierten Stosswellen zu einem feinen Gries zerkleinert, der auf natürliche Weise aus dem Körper herausgeschwemmt wird. Dabei sind die Vorrichtungen zur Erzeugung des Stosswellenfeldes und die Vorrichtungen zur Ortung so angebracht, dass sie sich räumlich nicht behindern. Die zwei Röntgengeräte zum stereoskopischen Orten sind neben dem Reflektor angeordnet. Die beiden Zentralstrahlen der zwei Röntgenortungsgeräte schneiden die Reflektorachse unter je einem Winkel von ca. 40° nahe dem Steinort. Diese Lösung mit zwei Röntgengeräten ist relativ aufwendig, ausserdem sind im Reflektor Ausschnitte für den Durchtritt der Röntgenstrahlung notwendig. Die im Bereich der Röntgenstrahlung liegenden Wasserstrecken beeinflussen die Aufnahmequalität oder machen das Einbringen von aufblasbaren Bälgen erforderlich.

Aufgabe der Erfindung ist es, eine Vorrichtung zum berührungsfreien Zerkleinern von Konkrementen in Körpern von Lebewesen zu schaffen, bei der die Ortung einen geringeren Aufwand erfordert und effizienter arbeitet.

Diese Aufgabe wird erfindungsgemäss von einer Vorrichtung mit den in den Ansprüchen genannten Merkmalen gelöst.

Mit der erfindungsgemässen Vorrichtung können die Geräte zur Ortung, zur Stosswellenerzeugung und zur Stosswellenfokussierung einfach ausgetauscht werden und liegen beim Betrieb in bezug auf den Patienten auf derselben Achse. Durch diese Massnahme erhält man mit einem einzigen Röntgensystem die x- und y-Koordinaten des Konkrements (des Steins). Die Röntgenstrecken sind sowohl im Körper wie auch im Wasser so klein wie möglich und entsprechen daher den geläufigen a-p (anteroposterior) Durchleuchtungs- und Aufnahmebedingungen. Damit sinken die Anforderungen an das einzige noch benötigte Röntgen-Ortungssystem gegenüber der bekannten Ausführung. Der Röntgenbildverstärker oder der Röntgenfilm befindet sich vorteilhafterweise möglichst nahe am Steinort, bei Nierensteinen unterhalb des Patienten, bei Gallensteinen oberhalb.

Die noch fehlende z-Koordinate des Konkrements (Entfernung von der Hautfläche) wird entweder vor der Behandlung durch eine Ultraschallmessung unter vergleichbaren Lagerungsbedingungen bestimmt oder es ist in die Mechanik ein Ultraschallsensor integriert, mit dem nach Einjustierung in x-y-Richtung die z-Koordinate bestimmt werden kann. Das Finden des Steins mit dem Ultraschallsensor ist deshalb gegenüber einem «blinden Suchen» dadurch wesentlich erleichtert, dass durch die vorausgehende Einjustierung in x-y-Richtung der Stein automatisch in der Schnittebene des Ultraschallsystems erscheint. Wird die x-y-Justierung mit dem Röntgensystem noch dazu benutzt, die Bewegungsrichtung des Steines infolge der Atembewegungen festzustellen und die Ultraschallebene parallel dazu auszurichten, so bleibt der Stein nach Austausch des Röntgensystems mit dem Ultraschallsystem im Ultraschallbild unabhängig von Atemexkursionen sichtbar. Die Tiefenmessung des Steines (z-Koordinate) kann unter Umständen dadurch ganz entfallen oder durch eine anatomische Schätzung ersetzt werden, dass statt des punkt- bis spindelförmigen Brennpunktes ein Linienfokus mit einer deutlich vergrösserten Ausdehnung in Achsenrichtung verwendet wird.

Die Erfindung kann sowohl bei einer Vorrichtung mit Wanne zum Ankoppeln der Stosswellen in den Körper als auch bei einer wannenfreien Vorrichtung verwendet werden.

Der erfindungsgemässe Ortswechsel zwischen Ortungs- und Stosswellensystem erfordert einen präzise und anatomisch optimal gelagerten Patienten, da der Austausch der Systeme beim wannenfreien System nicht berührungsfrei erfolgen kann und trotzdem keinerlei Verschiebungen des Patienten verursachen darf. Der Patient wird vorteilhaft in grossflächigen, anatomiegerecht geformten Schalen aufgenommen, die seitlich am Patienten möglichst weit hochgezogen sind. Die Schalen können mit passiv anformbaren Schichten (zum Beispiel Granulat unter Unterdruck) belegt sein, um eine stabile seitlich Führung des Patienten zu gewährleisten und durch eine möglichst kleine Flächenpressung die Durchblutungsverhältnisse in diesen Gebieten nicht zu verschlechtern.

In einer vorteilhaften Ausführung sind die Systeme zur Erzeugung und Fokussierung der Stosswellen und die Ortungssysteme auf zwei Schlitten über und unter dem Patienten befestigt, die senkrecht zur Körperachse des Patienten verschiebbar sind und in diskreten Stellungen einrasten.

Für eine Steinzerkleinerung ist es vorteilhaft, wenn sich das Stosswellenerzeugungs- und -führungssystem, zum Beispiel ein Ellipsoid mit Wasservorlaufstrecke zum Tiefenausgleich und Ankoppelmembran, die Röntgenstrahlungsquelle und der Ultraschallsensor an einem unter dem Patienten angeordneten und quer zu seiner Längsrichtung beweglichen Schlitten mit mehreren Arbeitsstellungen befinden. Der Röntgenbildverstärker oder die Röntgenfilmkassette und ein Auskoppelkissen (im Fall des wannenfreien Verfahrens) und gegebenenfalls ein weiterer Ultraschallsensor sind in einem Schlitten über dem Patienten angeordnet, der ebenfalls quer zur Längsachse des Patienten und mit dem unteren Schlitten koordiniert bewegt werden kann. Für die Nierensteinzerkleinerung ist es günstig, wenn sich der Röntgenbildverstärker nahe den Nieren, also unterhalb des Patienten befindet.

Die Justierverschiebungen des Patienten relativ zum Ellipsoid in Körperlängsrichtung (x-Richtung) und in der Höhe (z-Richtung) können vorteilhaft durch Verschiebungen der Patientenliege und die Justierung in y-Richtung durch Verschiebung der Einraststellen des unteren und oberen Geräteschlittens durchgeführt werden.

Die Verschiebeschlitten oberhalb und unterhalb des Patienten besitzen Vorrichtungen zum Einrasten in definierte Stellungen, zum Beispiel in drei Arbeitsstellungen in spielfrei ausgebildeten Klinken oder in ähnlichen Elementen. Zur seitlichen Positionierung (y-Richtung) kann das Klinkengehäuse und damit der Schlitten seitlich verfahren werden. Dadurch verschieben sich die Röntgen- und Applikationsachsen seitlich parallel, behalten aber ihre Zuordnung zueinander bei.

Die schlittenartigen Geräteträger können sowohl bei einem wannenfreien Verfahren als auch bei Verwendung einer Wanne eingesetzt werden.

Statt der linear beweglichen Schlitten können in beiden Fällen auch revolverartige Geräteträger Verwendung finden, bei denen der Platztausch der Geräte durch eine Drehbewegung erfolgt. Es können zwei Geräteträger für jeweils die oberen und die unteren Geräte verwendet werden. Die Drehachsen können entweder in Patientenlängsrichtung oder senkrecht dazu liegen. Eine mechanische oder elektrische Koordination der gegenseitigen Stellung kann vorgesehen werden.

In einer weiteren Ausführung sind alle Geräte auf einem einzigen kreisbogenförmigen Geräteträger angeordnet, der den Patienten umfasst und dessen Drehachse ungefähr mit der Patientenlängsachse zusammenfällt.

Bei Verwendung einer Wanne befindet sich im Boden unter der Nierengegend des Patienten ein röntgen- und stosswellendurchlässiges Fenster, das der Patient im allgemeinen nicht berührt, zum Beispiel eine 0,5 mm dicke Polyurethanfolie. Beim Ankoppeln der Stosswellen-Vorlaufstrecke wird sichergestellt, dass der Druck im Balgsystem der Vorlaufstrecke niedriger als der hydrostatische Druck am Boden der Wanne ist. Dies wird zum Beispiel durch ein einfaches Druckteilersystem erreicht, das den Balgdruck nach dem hydrostatischen Druck am Boden der Wanne steuert. Damit wird ein Hochwölben des Membranfensters und die Gefahr des Verschiebens des Patienten vermieden.

Dieses Prinzip findet auch Anwendung beim Einsatz als wannenfreies Verfahren, in dem ein dünnes Wasserkissen die Ankopplung übernimmt.

Vorteilhaft wird statt des punktförmigen Brennpunktes ein axialer Linienfokus verwendet. Dabei wird eine seitlich eng begrenzte, intensive Stosswellenfront über eine endliche Laufstrecke (Fokuslinie) zusammengehalten. Wegen des grösseren Wirkungsbereiches kann eine weniger aufwendige Ortung verwendet werden. Eine Fokusverbreiterung senkrecht zur Ausbreitungsrichtung der Stosswelle bei gleicher Druckamplitude im Fokus würde eine Erhöhung der Stosswellenenergie proportional zur Frontfläche erfordern und damit zu einer wesentlichen Erhöhung der Belastung von Elektroden und Patienten führen. Bei zu geringer Druckamplitude würde die «dynamische Dauerfestigkeit» des Steinmaterials unterschritten und damit keine Steinzerstörung mehr erreicht werden können.

Die Erfindung wird anhand von drei Figuren näher erläutert.

Es zeigen:

Fig. 1 eine Seitenansicht einer Ausführungsform einer erfindungsgemässen Vorrichtung,

Fig. 2 einen Querschnitt durch eine Ausführungsform,

Fig. 3 eine Draufsicht auf eine erfindungsgemässe Patientenliege.

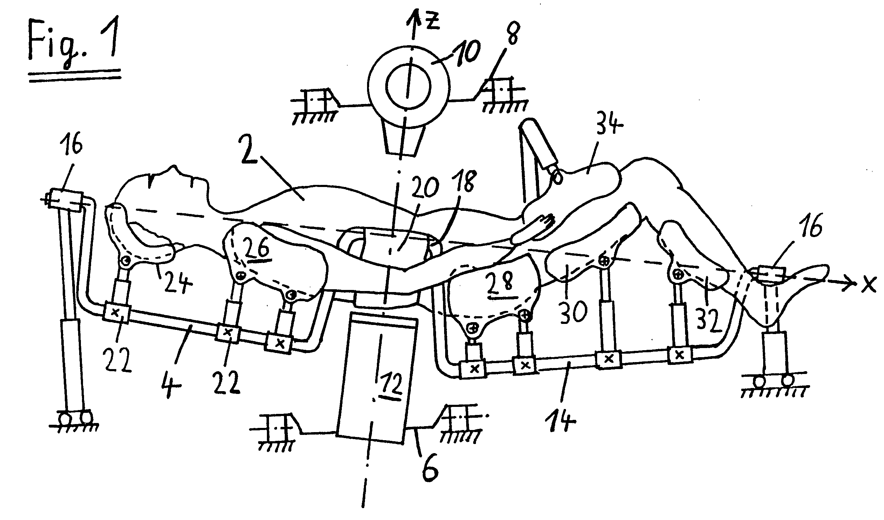

Fig. 1 zeigt eine Ausführung einer erfindungsgemässen Vorrichtung in Seitenansicht (x-, z-Ebene). Fig. 1 zeigt einen Patienten 2 auf einer Liege 4, einen Geräteträger (Schlitten) 6 unterhalb des Patienten 2 und einen Geräteträger (Schlitten) 8 oberhalb des Patienten 2.

Auf den Schlitten 6, 8 sind die Vorrichtungen zum Orten und zum Erzeugen, Fokussieren und Führen der Stosswellen angeordnet; oberhalb und unterhalb des Patienten (auf der z-Achse) befinden sich in dieser Figur am oberen Schlitten 8 eine Röntgenröhre 10 und am unteren Schlitten 6 ein Röntgenbildverstärker 12. Die Schlitten 6 und 8 sind senkrecht zur Zeichenebene (Richtung y) verschiebbar, nach einer Verschiebung um eine bestimmte Strecke nehmen dann die nicht gezeigten Ultraschall-Ortungsgeräte oder die Stosswellenquelle den Platz der Röntgenortungsgeräte 10, 12 am Patienten ein.

Die Patientenliege 4 ist modular aufgebaut, wobei möglichst gleichartige Teile (Schalen, Justiervorrichtungen) verwendet werden. Die Liege besteht hier aus einem Zentralrohr 14, das kopf- und fusswärts so gekröpft und gelagert ist (16), dass eine Drehung des Patienten 2 um seine Längsachse (x) möglich ist. Diese Drehbewegung ist normalerweise durch eine Bremse (nicht gezeigt) blokiert. Im Applikationsbereich ist das Zentralrohr 14 zweigeteilt (18) und etwa bis zur Mitte des Patienten 2 nach oben gekröpft. Der Zwischenraum zweichen den beiden Längsholmen im Mittelteil 18 wird entweder individuell eingestellt, oder stellt sich unter der Zugkraft der hängematteartig eingehängten, stosswellen- und röntgendurchlässigen Tragefolie 20 durch das Gewicht des Patienten 2 passiv ein. Auf reiterartigen, höhenverstellbaren Kulissensteinen 22 sind anatomisch geeignet geformte Tragschalen (zum Beispiel aus thermoplastischem Schalenmaterial, oder mit Vakuumkissen ausgekleidete Blechschalen) gelenkig, aber feststellbar angebracht. In dieser Ausführung bestehen sie aus Kopfschale 24, Rückenschale 26, Gesässschale 28, Oberschenkelschalen 30 und Unterschenkelschalen 32. Wird der Patient in einer Wanne gelagert (nicht gezeigt) können zum Abfangen des Auftriebs spezielle Niederhalter 34 oder Gurte (nicht gezeigt) verwendet werden.

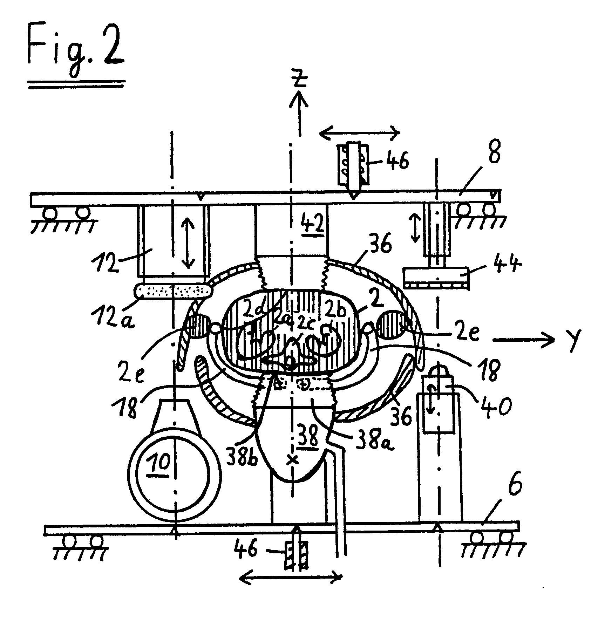

Fig. 2 zeigt eine Ausführung der erfindungsgemässen Vorrichtung für eine wannenfreie Steinzerkleinerung im Querschnitt (y-z-Ebene). Fig. 2 zeigt den Patientenkörper 2 mit der Nieren 2a, 2b, einen Rückenwirbel 2c, der Leber 2d und den Armen 2e, das Liegemittelteil 18, einen aus zwei Schalen 36 bestehenden aktiven oder passiven Wärmetunnel, der über den Patienten 2 geklappt oder geschoben werden kann und die zwei Geräteträger 6 und 8 mit den Ortungsgeräten und dem Stosswellensystem. Am unteren Schlitten 6 befinden sich die Röntgenröhre 10 der Stosswellenreflektor 38 und ein Ultraschallsensor 40. Am oberen Schlitten 8 befinden sich der Röntgenbildverstärker 12, ein Auskoppel- und Positionierkissen 42 und ein zweiter Ultraschallsensor 44.

Die beiden Schlitten 6, 8 rasten in drei Arbeitsstellungen in spielfrei ausgebildete Klinken 46 oder in äquivalente Geräte ein. Für unterschiedliche medizinische Aufgaben können auch mehrere Reflektoren (zum Beispiel für Nieren- und Gallensteine, Kinder und Erwachsene) auf den Trägern 6 und/oder 8 angebracht sein. Die Ankoppelstrecke 38a des Reflektors 38 ist seitlich so steif ausgebildet, dass sie die Tangentialkräfte, die durch das Koppelgel auf der Ankopplungsmembran 38b beim seitlichen Ausrücken entstehen, aufnehmen kann. Gegebenenfalls können entsprechende Führungselemente vorhanden sein.

Der Bildverstärker 12 ist in dieser Ausführung mit einem druckgesteuerten Luftkissen 12a an seiner Frontfläche versehen, das bei Berührung des Patienten die Hubbewegung beendet, damit der Patient nicht unter dem Einfluss der Zustellbewegung des Bildverstärkers nach unten gedrückt wird.

Die Ultraschallsensoren 40, 44 sind federnd so gelagert, dass sie sich automatisch luftspaltfrei an den mit Koppelgel versehenen Rücken (Bauch) des Patienten 2 anlegen.

Das Auskoppel- und Positionierkissen 42 ist wassergefüllt, dient zur Vermeidung von Verletzungen an der Auskoppelstelle und hält den Patientenkörper 2 in der vorgesehenen Lage. Der Wasserdruck im Positionierungskissen 42 wird vorteilhafterweise analog zum Anlegedruck der Ankoppelstrecke 38a des Ellipsoids 38 gesteuert, wodurch verhindert werden kann, dass der Patient 2 aus seiner Sollage gehoben wird. Wegen der leichteren Ankopplung und des kleineren Eintrittsfensters werden vorteilhafterweise Sektorscanner 40 verwendet werden.

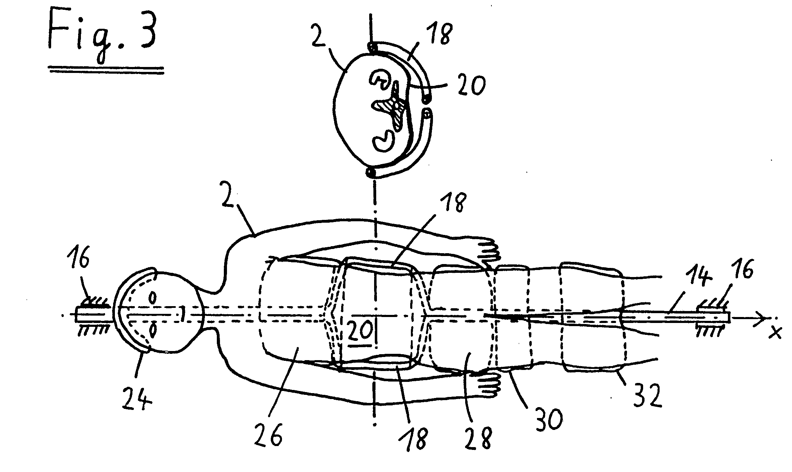

Fig. 3 zeigt einen Draufblick auf eine Ausführungsform einer Patientenliege, die der erfindungsgemässen Vorrichtung speziell angepasst ist. Zu erkennen ist die gekröpfte zweigeteilte Ausführung 18 des Zentralrohrs 14 im Applikationsbereich. Der Patientenkörper 2 wird dort von einer Tragefolie 20 unterstützt. Die anderen Körperteile werden von feststellbaren Trageschalen gehalten: Kopfschale 24, Rückenschale 26, Gesässschale 28, Oberschenkelschale 30, Unterschenkelschale 32 sowie nichtgezeigte Ellbogen-Unterarmschalen.

## Patentansprüche

1. Vorrichtung zur berührungsfreien Zerkleinerung von Konkrementen in Körpern (2) von Lebewesen mit einem Stosswellensystem, zum Beispiel Stosswellenquelle und Stosswellenreflektor (38), mindestens einem Ortungssystem (10, 12, 40, 44) und einer Patientenliege (4), dadurch gekennzeichnet, dass ein oder mehrere Geräteträger (6, 8) vorhanden sind, die einen Platztausch des Stosswellensystems (38, 42) mit dem oder den Ortungssystemen (10, 12, 40, 44) ermöglichen.

2. Vorrichtung nach Anspruch 1, dadurch gekennzeichnet, dass das Ortungssystem mindestens eine Röntgenquelle (10) und einen Röntgenbildverstärker (12) oder eine Röntgenfilmkassette umfasst.

3. Vorrichtung nach Anspruch 1 oder 2, dadurch gekennzeichnet, dass das Ortungssystem mindestens ein Ultraschall-Bilderzeugungssystem (40, 44) umfasst.

4. Vorrichtung nach einem der vorhergehenden Ansprüche, dadurch gekennzeichnet, dass die Liege (4) aus grossflächigen Schalen (24, 26, 28, 30, 32) besteht, die seitlich des Patienten hochgezogen und passiv anformbar sind und an einem Rohrrahmen verstellbar befestigt sind.

5. Vorrichtung nach einem der vorhergehenden Ansprüche, dadurch gekennzeichnet, dass der Geräteträger für Stosswellensystem und Ortungssystem als ein Schlitten ausgebildet ist, der senkrecht zur Körperachse des Patienten (2) verschiebbar ist und in diskreten Stellungen einrastet.

6. Vorrichtung nach Anspruch 5, dadurch gekennzeichnet, dass zwei parallel geführte Schlitten (6, 8) oberhalb und unterhalb des Patientenkörpers (2) vorhanden sind, wobei am oberen Schlitten (8) ein Röntgenbildverstärker (12) oder eine Röntgenfilmkassette und ein Ankoppelkissen (42) angeordnet sind und am unteren Schlitten (6) an den gegenüberliegenden Stellen eine Röntgenquelle (10), ein Reflektor (38) und ein Ultraschall-Ortungssystem (40) angeordnet sind.

7. Vorrichtung nach Anspruch 6, dadurch gekennzeichnet, dass sich die Röntgenquelle (10) oberhalb und der Röntgenbildverstärker (12) oder die Röntgenfilmkassette unterhalb des Patientenkörpers (2) befinden.

8. Vorrichtung nach einem der vorhergehenden Ansprüche, dadurch gekennzeichnet, dass der Patientenkörper (2) in einer Behandlungswanne gelagert ist, in deren Boden in der Nähe des Konkrements, zum Beispiel unter der Nierengegend des Patienten, ein röntgen-, ultraschall- und/oder stosswellendurchlässiges Fenster angebracht ist, das wahlweise durch seitliches und/oder senkrechtes Verschieben des unter dem Patienten angeordneten Geräteträgers, unterstützt durch eine Schicht von Koppelpaste, in flächenhaften, spaltfreien Kontakt mit den Ankoppelflächen des Röntgen-, Ultraschall- und/oder Stosswellensystems gebracht werden kann.

9. Vorrichtung nach einem der vorhergehenden Ansprüche, dadurch gekennzeichnet, dass über oder unter dem Patienten Wasserkissen vorgesehen sind, die den Patienten im Bereich des Abdomens und der Nierengegend so halten, dass das Konkrement durch das Ankoppeln von Geräten an das obere und untere Wasserkissen nicht aus dem Fokus bewegt wird.

10. Vorrichtung nach Anspruch 9, dadurch gekennzeichnet, dass an den Kissen zur Vermeidung von Verschiebungen des Patienten Vorrichtungen zum Druckausgleich vorgesehen sind.

11. Vorrichtung nach einem der vorhergehenden Ansprüche, dadurch gekennzeichnet, dass die Ge-

rätetråger als zwei drehbare Revolver jeweils für die Geräte über und unter dem Patienten ausgebildet sind und die Stellung der beiden Revolver mechanisch oder elektrisch koordiniert ist.

12. Vorrichtung nach einem der Ansprüche 1 bis 10, dadurch gekennzeichnet, dass ein revolverartiger, kreisbogenförmiger Geräteträger vorhanden ist, dessen Drehachse ungefähr mit der Patientenlängsachse zusammenfällt.

13. Vorrichtung nach einem der vorhergehenden Ansprüche, dadurch gekennzeichnet, dass verschiedene Reflektoren zur Behandlung von verschiedenen Konkrementen, wie Nierensteinen oder Gallensteinen, und zur Behandlung verschieden grosser Personen auf einem Geräteträger auswechselbar angeordnet sind.

14. Vorrichtung nach einem der vorhergehenden Ansprüche, dadurch gekennzeichnet, dass ein Stosswellensystem verwendet wird, das einen axialen Linienfokus erzeugt.

15. Vorrichtung nach einem der vorhergehenden Ansprüche, dadurch gekennzeichnet, dass die Geräte (40, 42, 44) federnd an den Geräteträgern (6, 8) aufgehängt sind und dass Vorrichtungen (12a, 38a) zum luftspaltfreien Anpassen an den Körper des Patienten vorgesehen sind.

## Claims

1. Apparatus for the contactless crushing of concretions in the bodies (2) of living beings, the apparatus having a shock wave system, for example a shock wave source and shock wave reflector (38), at least one locating system (10, 12, 40, 44) and a patients's bed (4), characterised in that one or more instrument carriers (6, 8) are fitted which provide for interchangeability of the shock wave system (38, 42) and the locating system or systems (10, 12, 40, 44).

2. Apparatus according to claim 1, characterised in that the locating system includes at least an X-ray source (10) and an X-ray image amplifier (12) or an X-ray film cassette.

3. Apparatus according to claim 1 or 2, characterised in that the locating system includes at least an ultrasonic image generating system (40, 44).

4. Apparatus according to one of the previous claims, characterised in that the bed (4) comprises shells having a large surface area (24, 16, 28, 30, 32) which can be raised at the side of the patient (2), which can be shaped by pressure, and which are attached to a tubular frame so that they can be adjusted.

5. Apparatus according to one of the previous claims, characterised in that the instrument carrier for the shock wave system and locating system is constructed as a slide which can be moved at right angles to the axis of the patient's body (2) and locked in discrete positions.

6. Apparatus according to claim 5, characterised in that two slides (6, 8) running parallel with each other are provided above and below the patient's (2) body, whereby an X-ray image amplifier (12) or an X-ray film cassette and a coupling pad (42) are arranged on the upper slide (8), whilst an X-ray source (10), a reflector (38) and an ultrasonic locating system (40) are arranged in the opposite positions on the lower slide (6).

7. Apparatus according to claim 6, characterised in that the X-ray source (10) is located above the patient's (2) body and the X-ray image amplifier (12) or the X-ray film cassette is located below the patient's (2) body.

8. Apparatus according to one of the previous claims, characterised in that the patient's (2) body is supported in a treatment bath, in the base of which, in the vicinity of the concretion, for example below the area of the patient's kidneys, an X-ray, ultrasonic, and/or shock wave transmitting window is fitted, which window can be brought at choice by lateral or vertical movements of the instruments carrier below the patient into surface contact with the coupling surfaces of the X-ray, ultrasonic and/or shock wave system without any gap and supported by a layer of coupling paste.

9. Apparatus according to one of the previous claims, characterised in that water cushions are provided above or below the patient which hold him in the area provided above or below the patient which hold him in the area of his abdomen and kidney in such a way that the concretion is not moved out of focus by the coupling of instruments at the upper and lower water cushion.

10. Apparatus according to claim 9, characterised in that pressure equalisation devices are provided at the cushions to prevent the patient from being moved.

11. Apparatus according to one of the previous claims, characterised in that instrument carriers are constructed as two rotatable turrets in each case for the instrument above and below the patient and the position of both turrets is coordinated mechanically or electrically.

12. Apparatus according to one of claims 1 to 10, characterised in that a turret-type, circular instrument carrier is provided, the axis of rotation of which coincides approximately with the longitudinal axis of the patient.

13. Apparatus according to one of the previous claims, characterised in that various reflectors for treating different concretions such as kidney stones or gall stones and for treating different sizes of patient are arranged interchangeably on an instrument carrier.

14. Apparatus according to one of the previous claims, characterised in that a shock wave system is used which generates an axial line focus.

15. Apparatus according to one of the previous claims, characterised in that the instruments (40, 42, 44) are spring mounted on the instrument carriers (6, 8) and that devices (12a, 38a) are provided for adapting the equipment to the body of the patient without air gaps.

## Revendications

1. Dispositif de morcellement sans contact de concrétions dans le corps (2) de personnes humaines, comprenant un système à ondes de choc, par exemple une source d'ondes de choc et un réflecteur d'ondes de choc (38), au moins un système de localisation (10, 12, 40, 44) et une couchette à patient (4), caractérisé en ce que l'on prévoit un ou plusieurs supports d'appareils (6, 8), qui permettent de remplacer le système à ondes de choc (38, 42) par le ou les systèmes de localisation (10, 12, 40, 44).

2. Dispositif selon la revendication 1, caractérisé en ce que le système de localisation comprend au moins une source de rayons X (10) et un amplificateur d'image de rayons X (12), ou une cassette à film Roentgen.

3. Dispositif selon la revendication 1 ou 2, caractérisé en ce que le système de localisation comprend au moins un système de formation d'images par ultrasons (40, 44).

4. Dispositif selon l'une quelconque des revendications précédentes, caractérisé en ce que la couchette (4) est constituée par des coquilles de grande surface (24, 26, 28, 30, 32), qui sont prolongées en hauteur sur les côtés du patient (2) et déformables passivement, et fixées de façon réglable à un cadre tubulaire.

5. Dispositif selon l'une quelconque des revendications précédentes, caractérisé en ce que le support d'appareils destiné au système à ondes de choc et au système de localisation est constitué sous forme d'un chariot qui peut être déplacé perpendiculairement à l'axe du corps de patient (2) et enclenché dans des positions discrètes.

6. Dispositif selon la revendication 5, caractérisé en ce que deux chariots guidés en parallèle (6, 8) sont prévus au-dessus et au-dessous du corps (2) du patient, un amplificateur d'images de rayons X (12) ou une cassette à film Roentgen et un coussin de couplage (42) étant disposés sur le chariot supérieur (2) et une source de rayons X (10), un réflecteur (38) et un système de localisation par ultrasons (40) étant disposés dans des positions opposées sur le chariot inférieur (6).

7. Dispositif selon la revendication 6, caractérisé en ce que la source de rayons X (10) se trouve au-dessus du corps (2) du patient et l'amplificateur d'images de rayons X (12) ou la cassette à film Roentgen se trouve au-dessous dudit corps.

8. Dispositif selon l'une quelconque des revendications précédentes, caractérisé en ce que le corps (2) du patient est disposé dans une baignoire de traitement dans le fond de laquelle est disposé à proximité de la concrétion, par exemple sous la région des reins du patient, une fenêtre perméable aux rayons X, aux ultrasons et/ou aux ondes de choc, qui peut être amenée au choix, par un déplacement latéral et/ou vertical du support d'appareils disposé au-dessous du patient, en étant renforcé par une couche de pâte de couplage, en contact de surface et sans interstice avec les surfaces de couplage du système à rayons X, ultrasons et/ou ondes de choc.

9. Dispositif selon l'une quelconque des revendications précédentes, caractérisé en ce que sont prévus au-dessus ou au-dessous du patient des coussins d'eau qui maintiennent le patient dans la région de l'abdomen et la région des reins de manière que la concrétion ne puisse pas être éloignée du foyer par le couplage d'appareils avec le coussin d'eau supérieur et le coussin d'eau inférieur.

10. Dispositif selon la revendication 9, caractérisé en ce que pour éviter des déplacements du patient, on prévoit sur les coussins des dispositifs d'équilibrage de la pression.

11. Dispositif selon l'une quelconque des revendications précédentes, caractérisé en ce que les supports d'appareils sont constitués sous forme de deux supports revolver tournants destinés respectivement aux appareils au-dessus et au-dessous du patient, et la position des deux supports revolver est coordonnée mécaniquement ou électriquement.

12. Dispositif selon l'une quelconque des revendications 1 à 10, caractérisé en ce qu'il est prévu un support d'appareils en forme d'arc de cercle et du type revolver, dont l'axe de rotation coïncide approximativement avec l'axe longitudinal du patient.

13. Dispositif selon l'une quelconque des revendications précédentes, caractérisé en ce que les divers réflecteurs destinés au traitement de concrétions diverses, telles que des calculs rénaux ou des calculs biliaires, et au traitement de personnes de tailles différentes, sont montés de façon à être remplacés les uns par les autres sur un support d'appareils.

14. Dispositif selon l'une quelconque des revendications précédentes, caractérisé en ce qu'on utilise un système à ondes de choc qui produit un foyer linéaire axial.

15. Dispositif selon l'une quelconque des revendications précédentes, caractérisé en ce que les appareils (40, 42, 44) sont suspendus élastiquement aux supports d'appareils (6, 8) et en ce que sont prévus des dispositifs (12a, 38a) pour réaliser une adaptation sans interstices d'air avec le corps du patient.

Fig. 1

# Fig.2

# Fig.3

0 166 102